(19) Europäisches Patentamt / European Patent Office / Office européen des brevets

(11) **EP 3 390 426 B1**

## (12) EUROPEAN PATENT SPECIFICATION

(45) Date of publication and mention
of the grant of the patent:
**26.06.2019 Bulletin 2019/26**

(21) Application number: **17705112.5**

(22) Date of filing: **14.02.2017**

(51) Int Cl.:
*C07K 7/06* (2006.01)      *A61K 39/395* (2006.01)
*C07K 19/00* (2006.01)      *C07K 14/47* (2006.01)
*C07K 14/435* (2006.01)      *C07K 16/18* (2006.01)
*A61K 47/64* (2017.01)      *A61K 38/03* (2006.01)
*A61K 38/17* (2006.01)      *A61P 25/28* (2006.01)

(86) International application number:
**PCT/EP2017/053242**

(87) International publication number:
**WO 2017/140656 (24.08.2017 Gazette 2017/34)**

(54) **AMYLOID CONJUGATE AND USES AND METHODS THEREOF**

AMYLOID-KONJUGAT SOWIE VERWENDUNGEN UND VERFAHREN DAFÜR

CONJUGUÉ D'AMYLOÏDE ET UTILISATIONS ET PROCÉDÉS ASSOCIÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.02.2016 ES 201630173**

(43) Date of publication of application:
**24.10.2018 Bulletin 2018/43**

(73) Proprietor: **Araclón Biotech, S. L.
50009 Zaragoza(Zaragoza) (ES)**

(72) Inventor: **SARASA BARRIO, Manuel
50009 Zaragoza (ES)**

(74) Representative: **Durán-Corretjer, S.L.P.
Còrsega, 329
(Paseo de Gracia/Diagonal)
08037 Barcelona (ES)**

(56) References cited:
WO-A1-2010/136487     WO-A1-2015/128287
WO-A2-2005/058940     WO-A2-2005/072777
WO-A2-2010/011947     US-A1- 2013 164 217
US-A1- 2013 230 545

- **GUO YIXIAN ET AL: "Potent antigen-specific immune response induced by infusion of spleen cells coupled with succinimidyl-4-(N-maleimidomethyl cyclohexane)-1-carboxylate (SMCC) conjugated antigens", INTERNATIONAL IMMUNOPHARMACOLOGY, ELSEVIER, AMSTERDAM, NL, vol. 31, 29 December 2015 (2015-12-29), pages 158-168, XP029412983, ISSN: 1567-5769, DOI: 10.1016/J.INTIMP.2015.12.023**
- **KAFI K ET AL: "Maleimide conjugation markedly enhances the immunogenicity of both human and murine idiotype-KLH vaccines", MOLECULAR IMMUNOLOGY, PERGAMON, GB, vol. 46, no. 3, 1 January 2009 (2009-01-01), pages 448-456, XP025816748, ISSN: 0161-5890, DOI: 10.1016/J.MOLIMM.2008.10.020 [retrieved on 2008-11-28]**
- **JOSEF KREJCI ET AL: "Effects of adjuvants on the immune response of pigs after intradermal administration of antigen", RESEARCH IN VETERINARY SCIENCE., vol. 94, no. 1, 1 February 2013 (2013-02-01), pages 73-76, XP055360955, GB ISSN: 0034-5288, DOI: 10.1016/j.rvsc.2012.07.021**

EP 3 390 426 B1

**Description**

**Field of the invention**

The present invention relates to the field of biochemistry, more specifically to the field of sector of protein conjugation. Additionally, the present invention has an application in the field of medicine and veterinary science in the treatment of amyloid diseases.

**Background of the invention**

Multiple conjugates useful for the active or passive immunization of patients with amyloid diseases, fundamentally for Alzheimer's disease, have been described in the prior art. It should be pointed out that most of said prior art focuses on selecting peptides or transport proteins that allow generating a suitable immune response in patients, the crosslinking agent used not being given much importance or relevance. Said crosslinking agent is often expressed in the form of a list of all those that are available or known up until now indicating that any of them can be used in an equivalent manner, or it is simply not even indicated.

For example, Spanish patent application with publication number ES2246105 discloses the prevention or treatment of amyloid diseases, *inter alia* Alzheimer's disease, by means of the active immunization of patients with the conjugate formed by the Aβ33-40 peptide and the transport protein keyhole limpet hemocyanin (hereinafter, KLH), with accession number 4BED in the Protein Data Bank. The use of said conjugate for generating antibodies (for example, by means of immunizing mammals or birds with said conjugate) which are subsequently used in a passive immunization method for the prevention or treatment of amyloid diseases, *inter alia* Alzheimer's disease, is also contemplated. Said patent document does not specify the crosslinking agent used.

The PCT patent application with publication number WO2005/072777 discloses conjugates for the active or passive immunization of patients based on the crosslinking agent LPA with respect to which the capacity thereof for binding with or having two peptides at the same time is highlighted, making it appropriate for generating an immune response suitable in patients. Within the general explanation of an LPA-based conjugate it is contemplated that the peptide used may be an Aβ42 or Aβ40 C-terminal fragment, and fragments 33-42, 35-42, 36-42, 37-42, 38-42 and 39-42 are specifically mentioned and exemplified. Additionally, said document mentions that the transport protein may be KLH. This same document describes generating other conjugates, using a different crosslinking agent (N-succinimidyl 3-(2-pyridyldithio) propionate, commonly known as SPDP).

Document US2013/230545 A1 discloses a pharmaceutical composition for use in the treatment or prevention of an amyloid disorder comprising conjugate of least one CysA.beta (33-40) peptide (SEQ ID NO: 1) linked to the keyhole limpet hemocyanin (KHL). Maleimidobutyric acid N-hydroxysuccinimide is not used as crosslinking agent.

Document WO2015/01282287 A1 discloses that a cysteine-containing peptide can be linked to KLH using the crosslinking agent maleimidobutyric acid N-hydroxysuccinimide. However, said document is silent about using aluminium hydroxide gel as adjuvant.

**Brief description of the invention**

After extensive and thorough experimentation, the inventors have surprisingly discovered that maleimidobutyric acid N-hydroxysuccinimide ester (hereinafter, SM), a heterobifunctional crosslinking agent, in which each molecule thereof binds a peptide to the transport protein, used as a crosslinking agent for preparing CysAβ(33-40) peptide (SEQ ID NO: 1) and KLH conjugates, produces conjugates that allow generating an improved immune response in comparison to the immune response produced by conjugates generated with other homo- or heterobifunctional crosslinking agents of the prior art such as SPDP which also allow the binding of a peptide to the transport protein. Such improvement is clearly shown in examples 1 to 4 of the present specification. In addition, said examples show that said new conjugates or the pharmaceutical compositions comprising said conjugates produce:

- an immune response that is as specific as possible, for the purpose of minimizing the side effects associated with the (preventive or therapeutic) vaccine treatment;

- the highest possible immune response for the purpose of assuring an effective immunization of patients and reducing the required doses of immunogenic conjugate.

Thus in a first aspect, the present invention relates to a composition comprising a conjugate of at least one CysAβ(33-40) peptide (SEQ ID NO: 1) linked to the keyhole limpet hemocyanin (KLH), wherein the crosslinking agent connecting each CysAβ(33-40) peptide to the keyhole limpet hemocyanin (KLH) of the conjugate is the maleimidobutyric acid N-hydroxysuccinimide ester (SM), wherein the composition further comprises aluminum hydroxide gel.

In a second aspect, the present invention relates to a pharmaceutical composition comprising i) a conjugate comprising at least one CysAβ(33-40) peptide (SEQ ID NO: 1) linked to the keyhole limpet hemocyanin (KLH) and ii) aluminum hydroxide gel, wherein the crosslinking agent connecting each CysAβ(33-40) peptide to the keyhole limpet hemocyanin (KLH) of the conjugate is the maleimidobutyric acid N-hydroxysuccinimide ester (SM), and wherein the pH of the composition ranges from 5.8 to 7.0.

Preferably, the above-mentioned pharmaceutical composition has a pH from 6.2 to 7.0. Also preferably, the pharmaceutical composition has a pH from 5.8 to 6.2.

Preferably, the above-mentioned pharmaceutical composition has an amount of CysAβ(33-40) peptides (SEQ ID NO: 1) at least 100 μg. Also preferably, the pharmaceutical composition has an amount of CysAβ(33-40) peptides (SEQ ID NO: 1) at least 150 μg. More preferably, the amount of CysAβ(33-40) peptides (SEQ ID NO: 1) in the pharmaceutical composition is from 150 μg to 400 μg. Yet more preferably, the amount of CysAβ(33-40) peptides (SEQ ID NO: 1) in the pharmaceutical composition is from 160 μg to 240 μg.

Preferably, the conjugates in the pharmaceutical composition in turn comprise a ratio of at least 45 CysAβ(33-40) peptides (SEQ ID NO: 1) linked to each keyhole limpet hemocyanin (KLH).

In another aspect, the present invention discloses a glass ampoule comprising the pharmaceutical composition as described above.

In a further aspect, the present invention relates to a method to manufacture the above-mentioned pharmaceutical composition, which comprises the following steps:

a. Adding maleimidobutyric acid N-hydroxysuccinimide ester (SM) to a solution comprising keyhole limpet hemocyanin (KLH) in a buffer at a pH between 7.0 to 9.0;
b. Eliminating the excess of maleimidobutyric acid N-hydroxysuccinimide ester from the solution of step a);
c. Adding CysAβ(33-40) peptides (SEQ ID NO: 1) in DMSO to the solution of step b) at a pH of between 6.6 to 7.0 to produce the conjugates;
d. Eliminating the free peptide from the solution from step c);
e. Optionally filtrating the solution of step d);
f. Adjusting the pH of the solution of step d) or e) to a pH range of between 5.8 to 6.2; and
g. Adding aluminum hydroxide gel to the solution of step f) once the pH has been adjusted.

Preferably, in said method the excess of of maleimidobutyric acid N-hydroxysuccinimide ester in step b) is eliminated by using 0.02 M Na-Phosphate-buffer at a pH of about 6.6 to 7.0 or by using 0.01 M PBS-buffer at a pH of 6.6 to 7.0.

Preferably, in the method of the present invention the solution is filtrated in step e) by using a filter of about 0.2 μm.

Preferably, in the method of the present invention the pH of step f) is adjusted to a pH range of about 6.0.

Preferably, the above-mentioned pharmaceutical or the glass ampoule are used in the treatment or prevention of an amyloid disease. More preferably, the amyloid disease is selected from the list consisting of Alzheimer's disease, Parkinson's disease, cerebral amyloid angiopathy, vascular dementia of an amyloid origin, inclusion-body myositis and dementia with Lewy bodies. Even more preferably, the amyloid disease is Alzheimer's disease.

## Detailed description of the invention

### Definitions

As used herein, "amyloid disease" and the plural thereof refer to diseases associated with the β-amyloid accumulation. Said accumulation can fundamentally occur in the brain, producing diseases among which are found Alzheimer's disease, Parkinson's disease, cerebral amyloid angiopathy, vascular dementia of an amyloid origin and dementia with Lewy bodies. β-amyloid accumulation can also fundamentally occur in skeletal muscle, producing inclusion-body myositis.

As used herein, "passive immunization" and the plural thereof refer to the delivery of antibodies or fragments thereof to a patient with the intention of conferring immunity to said patient.

As used herein, "active immunization" and the plural thereof refer to the delivery to a patient of peptides (in the form of conjugates) acting as immunogens, i.e., they allow antibody generation with the intention of conferring immunity to said patient.

As used herein, "adjuvant" and the plural thereof refer to immunomodulatory substances capable of being combined with the conjugate of the present invention for increasing, improving or otherwise modulating an immune response in a patient.

As used herein, "patient" and the plural thereof refer to any mammal, preferably human, in which the conjugate of the present invention or a composition comprising same can be administered for the purpose of treating or preventing an amyloid disease.

As used herein, "CysAβ(33-40)" refers to the sequence of positions 33 to 40 of Aβ40 (SEQ ID NO: 2) to which a cysteine has been added at the N-terminus. Said sequence is reflected in SEQ ID NO: 1 and is: CGLMVGGVV.

## Description

The invention refers to a pharmaceutical composition comprising i) a conjugate comprising at least one CysAβ(33-40) peptide (SEQ ID NO: 1) linked to the keyhole limpet hemocyanin (KLH) and ii) aluminum hydroxide gel, wherein the crosslinking agent connecting each CysAβ(33-40) peptide to the keyhole limpet hemocyanin (KLH) of the conjugate is the maleimidobutyric acid N-hydroxysuccinimide ester (SM).

It is noted that, as shown in example 5, the adsorption rates of the conjugate onto the aluminum hydroxide gel of said preferred composition depends on the pH. In fact, the adsorption rate increases at lower pH-values. A reduction from pH 7.4 to pH to 7.2 or even 7.0 does not result in significant higher adsorption rates. An adsorption at pH 6.8 shows an adsorption rate around 90%. At pH 6.0 the adsorption ratio is the highest. At a conjugate concentration based on 200 μg/mL net peptide and pH 6.7% of conjugate is still free.

Based on these analyses, in order to increase the immunogenic capacity of the pharmaceutical composition, a pH value of between 5.8 to 7.0 should be used in order to increase the adsorption rate of the conjugate onto the adjuvant and significantly increase its immunogenic capacity.

Therefore, another preferred embodiment of the second aspect of the invention refers to a composition, preferably a pharmaceutical composition, comprising i) a conjugate comprising at least one CysAβ(33-40) peptide (SEQ ID NO: 1) linked to the keyhole limpet hemocyanin (KLH) and ii) aluminum hydroxide gel, wherein the crosslinking agent connecting each CysAβ(33-40) peptide to the keyhole limpet hemocyanin (KLH) of the conjugate is the maleimidobutyric acid N-hydroxysuccinimide ester (SM), and wherein the pH of the composition ranges from 5.8 to 7.0, preferably from 6.2 to 7.0, more preferably from 5.8 to 6.2.

In further preferred embodiments of the second aspect of the invention or of any of its preferred embodiments, the amount of CysAβ(33-40) peptides (SEQ ID NO: 1) in the pharmaceutical composition is at least 100μg, preferably at least 150μg, more preferably from 150μg to 400μg, still more preferably from 160μg to 240μg, still more preferably about 200μg.

In yet further preferred embodiments of the second aspect of the invention or of any of its preferred embodiments, the KLH-SM-CysAβ(33-40) conjugates present in the pharmaceutical composition comprise a ratio of at least 45 CysAβ(33-40) peptides (SEQ ID NO: 1) per keyhole limpet hemocyanin (KLH) protein.

In still another preferred embodiment of the second aspect of the invention or of any of its preferred embodiments, the pharmaceutical composition in contained in glass ampoules, preferably of 1 or 1.2 ml.

It is further noted that, as reflected above, the adsorption rates of the conjugate onto the aluminum hydroxide gel of in the composition of the second aspect of the invention (when aluminum hydroxide gel is used) depends on the pH. Moreover, surprisingly the pH increases during the storage of the pharmaceutical composition once it has been manufactured thus reducing the adsorption rates if such increases reached pH values above 7.0, or preferably above 6.8. In order to diminish such drawback that clearly affects the immunogenic capacity of the pharmaceutical composition, it is important to adjust the pH of the pharmaceutical composition to a range of between 5.5 to 6.5, preferably 5.8 to 6.2, more preferably 5.9 to 6.1, at the time of manufacturing the pharmaceutical composition so that storage does not or affects minimally the immunogenic capacity of the composition.

Therefore, yet another preferred embodiment of the second aspect of the invention refers to a method to manufacture a pharmaceutical composition, which comprises the following steps:

> a. Adding maleimidobutyric acid N-hydroxysuccinimide ester (SM) to a solution comprising keyhole limpet hemocyanin (KLH) in a buffer at a pH between 7.0 to 9.0;
> b. Eliminating the excess of maleimidobutyric acid N-hydroxysuccinimide ester from the solution of step a), preferably by using 0,02 M Na-Phosphate-buffer at a pH of about 6.6 to 7.0;
> c. Adding CysAβ(33-40) peptides (SEQ ID NO: 1) in DMSO to the solution of step b) at a pH of between 6.6 to 7.0 to produce the conjugates;
> d. Eliminating the free peptide from the solution of step c), preferably by using 0,01 M PBS-buffer at a pH of 6,6 to 7.0;
> e. Optionally filtrating the solution of step d), preferably by using a filter of about 0.2 μm.;
> f. Adjusting the pH of the solution of step d) or e) to a pH range of between 5.5 to 6.5, preferably 5.8 to 6.2, more preferably 5.9 to 6.1, still more preferably about 6.0; and
> g. Adding aluminum hydroxide gel to the solution of step f) once the pH has been adjusted.

In a third aspect, the present invention discloses the use of a composition comprising the conjugate of the present invention for preparing a medicinal product. Preferably said composition is the composition identified in the second aspect of the invention or in any of its preferred embodiments. Also in a preferred embodiment, said medicinal product is for use in the treatment or prevention of an amyloid disease, more preferably of a amyloid disease selected from Alzheimer's disease, Parkinson's disease, cerebral amyloid angiopathy, vascular dementia of an amyloid origin, inclusion-body myositis and dementia with Lewy bodies. In the most preferred embodiment, said medicinal product is used for the prevention or treatment of Alzheimer's disease.

In a fourth aspect, the present invention relates to a method of treatment or of prevention of an amyloid disease in a patient in need of same comprising the delivery of a therapeutically effective amount of a composition comprising the conjugate of the present invention. Preferably said composition is the composition identified in the second aspect of the invention or in any of its preferred embodiments. Also in a preferred embodiment, said amyloid disease is a amyloid disease selected from Alzheimer's disease, Parkinson's disease, cerebral amyloid angiopathy, vascular dementia of an amyloid origin, inclusion-body myositis and dementia with Lewy bodies, even more preferably Alzheimer's disease.

In a final aspect, the present invention discloses a method of manufacturing antibodies characterized in that it comprises an immunization step for immunizing mammals or birds with a composition comprising the conjugate of the present invention. Preferably said composition is the composition identified in the second aspect of the invention or in any of its preferred embodiments. It is contemplated that the mammals used in such method can be ruminants, equidae, lagomorphs, primates (preferably humans) or any other mammal that allows obtaining the suitable amounts of serum for extracting or obtaining sufficient amounts of antibodies. It is contemplated that the birds used in the method of the present invention are any fowl-like birds, waterfowl, pigeons and doves or any other bird that allows obtaining suitable amounts of serum for extracting or obtaining sufficient amounts of antibodies. It is further contemplated the protection of the antibodies obtained or obtainable by the method of the final aspect of the invention as well as their use to manufacture a pharmaceutical composition for use in the treatment of an amyloid disease selected from Alzheimer's disease, Parkinson's disease, cerebral amyloid angiopathy, vascular dementia of an amyloid origin, inclusion-body myositis and dementia with Lewy bodies, even more preferably Alzheimer's disease.

Therefore, the present invention provides a conjugate generated using the crosslinking agent SM, and compositions comprising same that allow generating an greater immune response compared with conjugates generated with other crosslinking agents of the prior art.

Additionally, the immune response generated by said conjugates of the present invention or the compositions comprising said conjugates is specific for Aβ40, i.e., it allows generating anti-Aβ40 specific antibodies without generating anti-Aβ42 antibodies.

For better understanding, the present invention is described in further detail below in reference to the attached drawings presented by way of example and in reference to illustrative non-limiting examples.

**Example 1.** Preparation of KLH-SM-CysAβ(33-40) conjugates.

For preparing these conjugates, KLH was used as a transport protein, SM as a crosslinking agent and CysAβ(33-40) (SEQ ID NO: 1) as an immunogenic peptide (peptide with residues 33-40 of the amyloid peptide to which a cysteine has been added at the N-terminus).

Binding took place between the available lysine residues of the KLH and the cysteine added at the N-terminal end of the peptide. In this case, the binding of the crosslinking agent to KLH was done first (KLH activation step), and, in a second step, the immunogenic peptide was added to the activated KLH so that conjugation could take place.

The protocol followed for the foregoing is as follows:

- A 250 mM stock solution of SM was prepared by dissolving 100 mg of SM in 680 μL of dry DMSO. Aliquots of said stock solution were made and stored at -20°C.
- KLH was dissolved at a concentration of 5 mg/mL in PBS/5mM EDTA pH 7.4.
- 16 μL of stock solution of SM were added for each milliliter of KLH that was conjugated.
- Said solution was left to react for 2 hours and 30 minutes at room temperature under gentle stirring.
- Then, the reaction buffer was changed to remove reaction byproducts and excess unreacted SM. For this purpose, a PD10 column (GE Healthcare; reference 17-0851-01) was used as follows:

  ◦ Step a): The column was equilibrated with 25 mL (5 mL, 5 times) PBS (80 mM sodium hydrogenophosphate dihydrate, 20mM sodium dihydrogenophosphate monohydrate, 100mM sodium chloride) /5mM EDTA pH 7.4.
  ◦ Step b): 2.5 mL of the already reacted solution were added in the column and said solution was left to penetrate, discarding the eluate.
  ◦ Step c): 3.5 mL of PBS were added in the column and the eluate was collected.
  ◦ Step d): The column was re-equilibrated with 25 mL (5 mL, 5 times) of 5mM EDTA pH 7.4, as explained above in step a).

◦ Steps b) to d) were repeated all the times needed depending on the volume of solution that reacted. After that, the column was rendered equilibrated.

◦ Step e): The column was stored at 4°C in order to use it on other occasions with the same peptide conjugate in the same manner. Preceding protocol.

- Then the peptide that was to be mixed with activated KLH was calculated. To that end, was taken into account both the molecular weight of the peptide and the molecular weight of KLH, in addition to the active sites therein (measured according to the methods known in the prior art based on measuring absorbance at 343 nm before and after treating the activated KLH solution with dithiothreitol and performing the necessary conversion). For example, for 10 mg of KLH with 1724 active sites:

10/6725000 (mean molecular weight of KLH)= $1.48 \times 10^{-6}$ mmol of KLH

$1.48 \times 10^{-6}$ mmol of KLH x 1724 active sites= $2.55 \times 10^{-3}$ mmol of peptide required for covering all the active sites.

A 3-fold peptide excess was placed to favor the conjugation reaction:

$2.55 \times 10^{-3}$ x 3= $7.65 \times 10^{-3}$ mmol of peptide required.

$7.65 \times 10^{-3}$ x weight molecular of the peptide (834.4 Da)= mg of peptide required (6.38 mg of peptide) (applying the required conversion factors).

- Once the peptide/activated KLH ratio (i.e., KLH with bound SM) was determined, the following reaction was prepared: mixture of the peptide with the activated KLH with SM in a suitable proportion. To that end, the peptide was prepared at 6 mg/mL in DMSO and was slowly added to activated KLH. The proportion of DMSO in the final reaction must not exceed 30%. If it were higher than that in any case, PBS/5mM EDTA pH 7.4 is added until the amount thereof was reduced to values of less than 30%.
- The solution was left to react at room temperature and under stirring between 18 and 24 hours.
- Finally, the obtained solutions were stored at 4°C. The peptide that has not conjugated with KLH and is therefore free may or may not be removed. If said free peptide is not removed, the peptide concentration in the final product is determined by the total peptide used in the reaction, not only that which is bound to native KLH, and the final reaction volume.

**Example 2.** Comparison of the immune response of KLH-crosslinking agent-CisAβ(33-40) conjugates generated using different crosslinking agents.

In this case, the immune response strength generated in mice (4 per group) with the following conjugates was compared:

- KLH-SM-CysAβ(33-40) (produced according to Example 1).
- KLH-SPDP-CysAβ(33-40): this conjugate uses a crosslinking agent commonly used in the prior art, succinimidyl 3-(2-pyridyldithio)propionate (SPDP) and is produced by means of a protocol very similar to the one described in Example 1 (introducing the necessary adaptations) and known in the prior art.

The immune response strength test was conducted in BALB/c strain mice. The protocol that was followed was:

1. A week before the first inoculation, blood was drawn from all the mice participating in the study to obtain pre-immune serum.

2. Depending on the group to which each mouse was assigned (see Table 1 for the different analyzed groups), each mouse was inoculated the corresponding vaccine once a week for three weeks straight.

3. A week after the third shot, blood was drawn again from each of the mice participating in the study to measure the response obtained in the serum.

Table 1. Groups used in the study and description of the conjugate administered to each of them.

| Group | Transport protein | Crosslinking agent | Adjuvant |
|---|---|---|---|
| 1 | KLH, Manufacturer 1 | SM | Alhydrogel® (aluminum hydroxide gel) |
| 2 | KLH, Manufacturer 1 | SPDP | Alhydrogel® (aluminum hydroxide gel) |

(continued)

| Group | Transport protein | Crosslinking agent | Adjuvant |
|---|---|---|---|
| 3 | KLH, Manufacturer 2 | SM | Alhydrogel® (aluminum hydroxide gel) |
| 4 | KLH, Manufacturer 2 | SM | None |
| 5 | KLH, Manufacturer 2 | SPDP | Alhydrogel® (aluminum hydroxide gel) |
| 6 | KLH, Manufacturer 3 (GMP) | SM | Alhydrogel® (aluminum hydroxide gel) |

The peptide dose administered to the mice in each of the groups shown in Table 1 is reflected in Table 2 included below.

Table 2. Peptide dose, including both total peptide and peptide conjugated to KLH administered to each of the mice in each of the groups of the study.

| Group | Total peptide dose (sum of free peptide and KLH-bound peptide) (in μg) | KLH-bound peptide dose (in μg) |
|---|---|---|
| 1 | 120 | 40 |
| 2 | 120 | 40 |
| 3 | 120 | 40 |
| 4 | 120 | 40 |
| 5 | 120 | 40 |
| 6 | 60 | 60 |

Table 3 shows a tabulated summary of the immune response strength results obtained for the different groups (analyzing serum from the mice obtained the week after finishing the therapeutic or vaccination regimen explained in the present example), together with the increase observed in the immune response (fold number the immune response increased a week after finishing the therapeutic regimen with respect to the pre-immune response). The measurement of the immune response was taken on the serum obtained from each mouse by means of indirect ELISA, according to the protocol known in the prior art, with respect to which it should be pointed out that the ELISA plates were sieved with $A\beta40$ peptide. Once the corresponding steps of washing, blocking, subsequent washing, incubation with the samples to be analyzed of plasma/serum (1:3 serial dilutions starting with a 1:30 dilution) and additional washing were performed, each well was incubated with the Anti-mouse IgG (H+L) antibody HRP (the secondary antibody dilution was 1:2000 in vehicle solution at pH 8). After incubating with said antibody and washing the wells, the plate was developed by adding 100 μL per well of an ABTS solution (diammonium 2,2'-azinobis-[3-ethylbenzothiazolinesulfonate]; Roche; reference: 102 946 001) with 0.375 mg/mL in ABTS buffer (Roche; reference: 11 112 597 001). This substrate turned green upon reacting with the peroxidase bound to the secondary antibody. The color intensity depended on the amount of antibodies bound to the plate. The reaction was incubated for 55 minutes at room temperature and in the dark, and then the absorbance was read in an ELISA plates reader at 405 nm. The obtained absorbance results were analyzed with the GraphPad Prism 3.02 program. The "One Site Competition" equation was used for the analysis:

$$Y = Minimum + \frac{(Maximum - Minimum)}{1 + 10^{x - LogEC50}}$$

The $EC_{50}$ data, which is the inflection point of the curve, i.e., the point at which 50% of the maximum effect observed was produced, was obtained from the aforementioned analysis. In the present case, it was interpreted as the serum dilution at which 50% of the peptide present in the well bound to the antibody present in the serum.

Table 3. Mean immune response results obtained for each of the groups of the study. It includes results for the serum after the vaccine treatment (1 week after the three injections according to the protocol described in the present example) and the increase observed between said point and the pre-immune response (before starting the therapeutic regimen). The mean pre-immune $EC_{50}$ was 18.11.

| Group | Post-3 inoculation $EC_{50}$ | Increase in $EC_{50}$ (fold number) |
|---|---|---|
| 1 | 12974.95 | 716.33 |
| 2 | 580.35 | 32.04 |
| 3 | 6325.50 | 349.22 |
| 4 | 924.97 | 51.07 |
| 5 | 1.87 | 0.10 |
| 6 | 5056.00 | 279.14 |

In view of the results shown in Table 3 and the conjugate used in each of the study groups, the following can be concluded:

- The different KLHs used as transport proteins (from different manufacturers), despite having a certain effect on the immune response, have not been shown to be relevant in determining the magnitude of the immune response (high immune response in groups 1, 3, 4 and 6, i.e., groups with an increase in $EC_{50}$ of 50 or more, versus a weak or inexistent immune response in groups 2 and 5, i.e., groups with an increase in $EC_{50}$ of less than 50).
- The differences between obtaining a high immune response and a weak or inexistent immune response lie in the crosslinking agent used. Based on the experimental results obtained, it is deduced that the conjugates in which SM were used produce an immune response that is significantly greater that the one observed for the conjugates in which SPDP was used. In fact, the groups treated with conjugates with the crosslinking agent SM allow generating a high immune response whereas the groups treated with conjugates in which SPDP was used showed an immune response that was much weaker or inexistent.
- In addition to the foregoing, the results obtained for group 4 must be highlighted, as they clearly show that the response of the conjugates generated using SM is still greater than the one observed for the conjugates generated using SPDP, even without the use of an adjuvant.

The foregoing shows that the use of SM as a crosslinking agent for preparing the KLH-crosslinking agent-CysAβ(33-40) conjugates provides vaccines with an immune response that is surprisingly greater whether said conjugate is used with an adjuvant or without an adjuvant.

**Example 3.** Comparison of the degree of conjugation (peptide binding to KLH) of KLH-crosslinking agent-CysAβ(33-40) conjugates generated using different crosslinking agents.

As in the case of Example 2, the conjugates for which the degree of conjugation or number of peptides bound per molecule of transport protein (KLH) was compared are:

- KLH-SM-CysAβ(33-40) (produced according to Example 1).
- KLH-SPDP-CysAβ(33-40) (produced as indicated in Example 2).

Table 4 shows a tabulated summary of the experimental results obtained for the experiment for binding the peptide to the transport protein that was carried out.

Table 4. Description of the analyzed conjugates and of the number of peptide molecules bound to each KLH molecule observed by mass spectrometry. With respect to said bonds, the table indicates one or two values depending on if one or two batches of the corresponding conjugate were analyzed.

| Transport protein | Crosslinking agent | Number of peptide molecules bound to each KLH molecule |
|---|---|---|
| KLH, Manufacturer 1 | SM | 81 |
| KLH, Manufacturer 1 | SPDP | 35/33 |

(continued)

| Transport protein | Crosslinking agent | Number of peptide molecules bound to each KLH molecule |
|---|---|---|
| KLH, Manufacturer 2 | SM | 70/68 |
| KLH, Manufacturer 2 | SPDP | 44/31 |

As seen in Table 4, those KLHs from different manufacturers had no effect on in the bonds result obtained. In contrast, the crosslinking agent did indeed have an enormous effect on the results obtained given that SM allowed obtaining twice the number of bonds or more, i.e., by using SM as a crosslinking agent twice the number of peptide molecules binds for each KLH molecule. This result is surprising and unexpected given that the peptide used incorporates a cysteine at the N-terminus for reacting with the crosslinking agents. According to the prior art, the incorporation of said cysteine should allow the peptide conjugation to be efficient and equivalent to any of the crosslinking agents known in the prior art. However, it was observed in this case that SM allows a more efficient conjugation reaction than SPDP does.

These binding results allowed explaining part of the results shown in Example 2 (i.e., part of the improvement observed in immune response induction and subsequent antibody generation). Nevertheless, said immune response results are not completely assimilable to the obtained binding results, which is also surprising in view of said obtained results and suggesting that the crosslinking agent contributes to increasing the immune response not only by mediating greater binding of the peptide to the transport protein.

**Example 4.** Immune response assays in rabbits and specificity of the antibodies generated.

A potency assay of vaccines comprising the KLH-SM-CysA$\beta$(33-40) conjugates was conducted in rabbits. In this case, the rabbits were vaccinated with 200 $\mu$g of total bound peptide, said 200 $\mu$g being bound to the transport protein (chosen dose depending on preliminary studies). Each rabbit was inoculated with 1 mL of the vaccine with the previously specified dose, using 2% Alhydrogel® (aluminum hydroxide gel) as an adjuvant.

The animals were treated with the previously indicated dose by means of subcutaneous injection of the vaccine once a week for 3 straight weeks drawing blood a week before commencing the vaccination protocol and a week after finishing it.

The titration of the generated antibodies and the analysis of their specificity was done by means of ELISA, according to the protocol known in the prior art and briefly indicated in Example 2, with the following differences:

- The ELISA plates were sieved with A$\beta$40 or A$\beta$42 peptide (included as SEQ ID NO: 3), depending on if specific antibodies are to be detected or those that bind to A$\beta$40 or to A$\beta$42, respectively.
- The antibody used to detect the presence of antibodies in the analyzed sera was Anti-Rabbit IgG (H+L) HRP (Invitrogen; reference: 65-6120) (the secondary antibody dilution was 1:2000 in vehicle solution at pH 8).

The development reagents were those indicated in Example 2, and therefore the plates were also read at 405 nm and the same equation was applied to the results. The $EC_{50}$ data, which is the inflection point of the curve, i.e., the point at which 50% of the maximum effect observed was produced, was obtained from the analysis. As indicated in Example 2, said result was interpreted as the serum dilution at which 50% of the peptide present in the well bound to the antibody present in the serum.

According to the aforementioned titration protocol, all the samples obtained were titrated to detect A$\beta$40 and A$\beta$42 peptide antibodies. The obtained results are shown in tabulated form in Tables 5 and 6.

Table 5. Amount of anti-A$\beta$40 specific antibodies in rabbits before and after the vaccination protocol. A column relating to the increase observed as a consequence of treatment is also included.

| Identification of Rabbit | Pre-immune $EC_{50}$ | Post-3 inoculation $EC_{50}$ | Increase in $EC_{50}$ (fold number) |
|---|---|---|---|
| 71 | 36.17 | 47406 | 1310.64 |
| 72 | 7.483 | 4021 | 537.35 |
| 73 | 14.07 | 73421 | 5218.27 |
| 74 | 4.482 | 12467 | 2781.57 |
| 75 | 45.48 | 5173 | 113.74 |

(continued)

| Identification of Rabbit | Pre-immune EC$_{50}$ | Post-3 inoculation EC$_{50}$ | Increase in EC$_{50}$ (fold number) |
|---|---|---|---|
| 76 | 20.5 | 98381 | 4799.07 |

Table 6. Amount of Aβ42 specific antibodies in rabbits before and after the vaccination protocol. A column relating to the increase observed as a consequence of treatment is also included.

| Identification of Rabbit | Pre-immune EC$_{50}$ | Post-3 inoculation EC$_{50}$ | Increase in EC$_{50}$ (fold number) |
|---|---|---|---|
| 71 | 53.88 | 0.049 | 0.00 |
| 72 | 29.24 | 0.069 | 0.00 |
| 73 | 22.49 | 2.761 | 0.12 |
| 74 | 24.05 | 15.49 | 0.64 |
| 75 | 73.18 | 0.063 | 0.00 |
| 76 | 23.37 | 34.04 | 1.46 |

Based on what is shown in Tables 5 and 6, it is deduced that the conjugate of the present invention (KLH-SM-CysAβ(33-40)) allows not only obtaining a high immune response in rabbits but also said response is specific for Aβ40 (without a significant humoral response to Aβ42), i.e., anti-Aβ40 specific antibodies are generated in the immune response that do not bind to Aβ42.

The results included in Examples 1 to 4 prove and confirm the technical advantages and effects explained above in the description, proving that the use of SM as a crosslinking agent allows generating KLH-crosslinking agent-CysAβ(33-40) conjugates that generate a greater immune response with respect to when another crosslinking agent of the prior art is used. Additionally, KLH-SM-CisAβ(33-40) conjugates allow generating high immune responses in mice and rabbits, specific for Aβ40 (without a significant humoral response to Aβ42), i.e., anti-Aβ40 specific antibodies that do not bind to Aβ42 are generated in said immune responses. Said examples validate the usefulness of the conjugate of the present invention in the treatment of amyloid diseases, preferably Alzheimer's disease, in mammals, preferably in humans.

**Example 5.** Adsorption studies for KLH-SM-CysAβ(33-40) conjugates onto the aluminum hydroxide gel.

1.35 mg/ml, peptide is equivalent to 14.4 mg/ml conjugate KLH-SM-CysAβ(33-40).

Tables 7.

| μg/mL based on peptide net | μg/mL peptide net supernatant | equivalent to Abeta-X40-KLH supernatant [mg/mL] | % conjugate free in solution | % adsorbed |
|---|---|---|---|---|
| 100 | 12 | 0,128 | 12 | 88 |
| 125 | 15 | 0,160 | 12 | 88 |
| 150 | 19 | 0,203 | 13 | 87 |
| 175 | 25 | 0,267 | 14 | 86 |
| 200 | 35 | 0,373 | 18 | 83 |
| **Study 2:** | **Adsorption-dependency of pH at defined concentrations** | | | |
| **Testing 1:** | **220 μg peptide net/mL** | | | |
| pH | μg/mL peptide net supernatant | equivalent to Abeta-X40-KLH supernatant [mg/mL] | % conjugate free in solution | % adsorbed |
| 7,4 | 38 | 0,41 | 17 | 83 |

(continued)

| Study 2: | Adsorption-dependency of pH at defined concentrations | | | |
|---|---|---|---|---|
| Testing 1: | 220 μg peptide net/mL | | | |
| pH | μg/mL peptide net supernatant | equivalent to Abeta-X40-KLH supernatant [mg/mL] | % conjugate free in solution | % adsorbed |
| 7,2 | 37 | 0,39 | 17 | 83 |
| 7,0 | 33 | 0,35 | 15 | 85 |
| 6,8 | 25 | 0,27 | 11 | 89 |
| 6,5 | 21 | 0,22 | 10 | 90 |
| 6,0 | 15 | 0,16 | 7 | 93 |
| Testing | 150 μg peptide net/mL | | | |
| pH | μ/mL peptide net supernatant | equivalent to Abeta-X40-KLH supernatant [mg/mL] | % conjugate free in solution | % adsorbed |
| 7,4 | 18 | 0,19 | 12 | 88 |
| 7,2 | 18 | 0,19 | 12 | 88 |
| 7,0 | 15 | 0,16 | 10 | 90 |
| 6,8 | 10 | 0,11 | 7 | 93 |
| 6,5 | 11 | 0,12 | 7 | 93 |
| 6,0 | 0 | 0,00 | 0 | 100 |
| Testing 3: | 100 μg peptide net/mL | | | |
| pH | μg/mL peptide net supernatant | equivalent to Abeta-40-KLH supernatant [mg/mL] | % conjugate free in solution | % adsorbed |
| 7,4 | 13 | 0,14 | 13 | 87 |
| 7,2 | 13 | 0,14 | 13 | 87 |
| 7,0 | 12 | 0,13 | 12 | 88 |
| 6,8 | 9 | 0,10 | 9 | 91 |
| 6,5 | 0 | 0,00 | 0 | 100 |
| 6,0 | 0 | 0,00 | 0 | 100 |

In these studies the influence of the concentration of conjugate on the adsorption rate as well as the influence of the pH on the adsorption rate were determined. In the first experiment the adsorption rate of different concentrations of conjugate in a defined amount of aluminium hydroxide (0.35 % corresponds to the permitted dose of 1.25 mg Al per single dose) and the defined pH 7.4 (physiological pH) was tested. At the physiological testing conditions (pH 7.4) 12 to 18 % free conjugate was detected in the supernatant. A reduction of the amount of peptide to 100 μg peptide does not yield higher adsorption rates.

In a second study the influence of the pH on the adsorption rate was determined. The pH was altered (pH 6.0 to pH 7.4) at defined conjugate concentrations (based on 100 μg, 150 μg and 220 μg peptide net/mL). As illustrated in the results shown above, the adsorption rate depends on the pH. In fact, the adsorption rate increases at lower pH-values (see tables): A reduction from pH 7.4 to pH to 7.2 or even 7.0 does not result in significant higher adsorption rates. An adsorption at pH 6.8 shows an adsorption rate around 90 %. At pH 6.0 the adsorption ratio is the highest. At a conjugate concentration based on 200 μg/mL net peptide and pH 6, 7% of conjugate is still free.

Based on these analyses, in order to increase the immunogenic capacity of the pharmaceutical composition of the invention, a pH value of between 5.8 to 7.0 should preferably be used in order to increase the adsorption rate of the conjugate onto the adjuvant.

Although the invention has been described with respect to preferred embodiments, the latter must not be considered to be limiting of the invention, which will be defined by the broadest interpretation of the following claims.

**Claims**

1. A composition comprising a conjugate of at least one CysAβ(33-40) peptide (SEQ ID NO: 1) linked to the keyhole limpet hemocyanin (KLH), wherein the crosslinking agent connecting each CysAβ(33-40) peptide to the keyhole limpet hemocyanin (KLH) of the conjugate is the maleimidobutyric acid N-hydroxysuccinimide ester (SM), wherein the composition further comprises aluminum hydroxide gel.

2. A pharmaceutical composition comprising i) a conjugate comprising at least one CysAβ(33-40) peptide (SEQ ID NO: 1) linked to the keyhole limpet hemocyanin (KLH) and ii) aluminum hydroxide gel, wherein the crosslinking agent connecting each CysAβ(33-40) peptide to the keyhole limpet hemocyanin (KLH) of the conjugate is the maleimidobutyric acid N-hydroxysuccinimide ester (SM), and wherein the pH of the composition ranges from 5.8 to 7.0.

3. The pharmaceutical composition according to claim 2, wherein the pH of the composition ranges from 6.2 to 7.0.

4. The pharmaceutical composition according to claim 2, wherein the pH of the composition ranges from 5.8 to 6.2.

5. The pharmaceutical composition according to any of claims 2 to 4, wherein the amount of CysAβ(33-40) peptides (SEQ ID NO: 1) in the pharmaceutical composition is at least 100μg.

6. The pharmaceutical composition according to any of claims 2 to 4, wherein the amount of CysAβ(33-40) peptides (SEQ ID NO: 1) in the pharmaceutical composition is at least 150μg.

7. The pharmaceutical composition according to any of claims 2 to 4, wherein the amount of CysAβ(33-40) peptides (SEQ ID NO: 1) in the pharmaceutical composition is from 150μg to 400μg.

8. The pharmaceutical composition according to any of claims 2 to 4, wherein the amount of CysAβ(33-40) peptides (SEQ ID NO: 1) in the pharmaceutical composition is from 160μg to 240μg.

9. The pharmaceutical composition according to any of claims 2 to 8, wherein the conjugates in turn comprise a ratio of at least 45 CysAβ(33-40) peptides (SEQ ID NO: 1) linked to each keyhole limpet hemocyanin (KLH).

10. A glass ampoule comprising the pharmaceutical composition of any of claims 2 to 9.

11. A method to manufacture a pharmaceutical composition according to any of claims 2 to 9, which comprises the following steps:

   a. Adding maleimidobutyric acid N-hydroxysuccinimide ester (SM) to a solution comprising keyhole limpet hemocyanin (KLH) in a buffer at a pH between 7.0 to 9.0;
   b. Eliminating the excess of maleimidobutyric acid N-hydroxysuccinimide ester from the solution of step a);
   c. Adding CysAβ(33-40) peptides (SEQ ID NO: 1) in DMSO to the solution of step b) at a pH of between 6.6 to 7.0 to produce the conjugates;
   d. Eliminating the free peptide from the solution from step c);
   e. Optionally filtrating the solution of step d);
   f. Adjusting the pH of the solution of step d) or e) to a pH range of between 5.8 to 6.2; and
   g. Adding aluminum hydroxide gel to the solution of step f) once the pH has been adjusted.

12. The method of claim 11, wherein the excess of step b) is eliminated by using 0.02 M Na-Phosphate-buffer at a pH of 6.6 to 7.0.

13. The method of any of claims 11 or 12, wherein the excess of step d) is eliminated by using 0.01 M PBS-buffer at a pH of 6.6 to 7.0.

14. The method of any of claims 11 to 13, wherein the solution is filtrated in step e) by using a filter of 0.2 μm.

15. The method of any of claims 11 to 14, wherein the pH of step f) is adjusted to a pH range of 6.0.

16. The pharmaceutical composition of any of claims 2 to 9 or the glass ampoule of claim 10, for use in the treatment

EP 3 390 426 B1

or prevention of an amyloid disease.

17. The pharmaceutical composition for use of claim 16, wherein the amyloid disease is selected from the list consisting of Alzheimer's disease, Parkinson's disease, cerebral amyloid angiopathy, vascular dementia of an amyloid origin, inclusion-body myositis and dementia with Lewy bodies.

18. The pharmaceutical composition for use of claim 17, wherein the amyloid disease is Alzheimer's disease.

**Patentansprüche**

1. Zusammensetzung, umfassend ein Konjugat aus mindestens einem CysAβ(33-40)-Peptid (SEQ ID NO: 1), das mit dem Napfschnecken-Hämocyanin (KLH) für Englisch: keyhole limpet hemocanin) verbunden ist, wobei das Vernetzungsmittel, das jedes CysAβ(33-40)-Peptid mit dem Napfschnecken-Hämocyanin (KLH) des Konjugats verbindet, der Maleinimidbuttersäure-N-Hydroxysuccinimidester (SM) ist, wobei die Zusammensetzung ferner Aluminiumhydroxidgel umfasst.

2. Pharmazeutische Zusammensetzung, umfassend i) ein Konjugat, umfassend mindestens ein CysAβ(33-40)-Peptid (SEQ ID NO: 1), das mit dem Napfschnecken-Hämocyanin (KLH) verbunden ist, und ii) Aluminiumhydroxidgel, wobei das Vernetzungsmittel, das jedes CysAß(33-40)-Peptid mit dem Napfschnecken-Hämocyanin (KLH) des Konjugats verbindet, der Maleimidbuttersäure-N-hydroxysuccinimidester (SM) ist, und wobei der pH-Wert der Zusammensetzung von 5,8 bis 7,0 reicht.

3. Pharmazeutische Zusammensetzung nach Anspruch 2, wobei der pH-Wert der Zusammensetzung im Bereich von 6,2 bis 7,0 liegt.

4. Pharmazeutische Zusammensetzung nach Anspruch 2, wobei der pH-Wert der Zusammensetzung im Bereich von 5,8 bis 6,2 liegt.

5. Pharmazeutische Zusammensetzung nach einem der Ansprüche 2 bis 4, wobei die Menge an CysAβ(33-40)-Peptiden (SEQ ID NO: 1) in der pharmazeutischen Zusammensetzung mindestens 100 μg beträgt.

6. Pharmazeutische Zusammensetzung nach einem der Ansprüche 2 bis 4, wobei die Menge an CysAβ(33-40)-Peptiden (SEQ ID NO: 1) in der pharmazeutischen Zusammensetzung mindestens 150 μg beträgt.

7. Pharmazeutische Zusammensetzung nach einem der Ansprüche 2 bis 4, wobei die Menge an CysAβ(33-40)-Peptiden (SEQ ID NO: 1) in der pharmazeutischen Zusammensetzung von 150 μg bis 400 μg beträgt.

8. Pharmazeutische Zusammensetzung nach einem der Ansprüche 2 bis 4, wobei die Menge an CysAβ(33-40)-Peptiden (SEQ ID NO: 1) in der pharmazeutischen Zusammensetzung von 160 μg bis 240 μg beträgt.

9. Pharmazeutische Zusammensetzung nach einem der Ansprüche 2 bis 8, wobei die Konjugate wiederum ein Verhältnis von mindestens 45 CysAβ(33-40)-Peptiden (SEQ ID NO: 1), die mit jedem Napfschnecken-Hemocyanin (KLH) verbunden sind, umfassen.

10. Glasampulle, umfassend die pharmazeutische Zusammensetzung nach einem der Ansprüche 2 bis 9.

11. Verfahren zur Herstellung einer pharmazeutischen Zusammensetzung nach einem der Ansprüche 2 bis 9, umfassend die folgenden Schritte:

a) Zugeben von Maleimidbuttersäure-N-hydroxysuccinimidester (SM) zu einer Lösung, die Napfschnecken-Hämocyanin (KLH) in einem Puffer mit einem pH-Wert zwischen 7,0 bis 9,0 umfasst;
b) Eliminieren des Überschusses an Maleimidbuttersäure-N-hydroxysuccinimidester aus der Lösung des Schritts a);
c) Zugeben von CysAβ(33-40)-Peptiden (SEQ ID NO: 1) in DMSO zu der Lösung des Schritts b) bei einem pH-Wert zwischen 6,6 bis 7,0, um die Konjugate herzustellen;
d) Eliminierung des freien Peptids aus der Lösung des Schritts c);
e) optionales Filtrieren der Lösung des Schritts d);

14

f) Einstellen des pH-Wertes der Lösung des Schritts d) oder e) auf einen pH-Bereich zwischen 5,8 bis 6,2; und
g) Zugeben von Aluminiumhydroxidgel zu der Lösung des Schritts f), sobald der pH-Wert eingestellt wurde.

12. Verfahren nach Anspruch 11, wobei der Überschuss von Schritt b) unter Verwendung von 0,02 M Na-Phosphat-Puffer bei einem pH-Wert von etwa 6,6 bis 7,0 eliminiert wird.

13. Verfahren nach einem der Ansprüche 11 oder 12, wobei der Überschuss des Schritts d) durch Verwendung von 0,01 M PBS-Puffer mit einem pH-Wert von 6,6 bis 7,0 eliminiert wird.

14. Verfahren nach einem der Ansprüche 11 bis 13, wobei die Lösung in dem Schritt e) unter Verwendung eines Filters mit 0,2 $\mu$m filtriert wird.

15. Verfahren nach einem der Ansprüche 11 bis 14, wobei der pH-Wert des Schritts f) auf einen pH-Bereich von 6,0 eingestellt wird.

16. Pharmazeutische Zusammensetzung nach einem der Ansprüche 2 bis 9 oder der Glasampulle nach Anspruch 10 zur Verwendung bei der Behandlung oder Prävention einer Amyloidkrankheit.

17. Pharmazeutische Zusammensetzung zur Verwendung nach Anspruch 16, wobei die Amyloidkrankheit aus der Liste ausgewählt ist, bestehend aus Alzheimer-Krankheit, Parkinson-Krankheit, zerebraler Amyloid-Angiopathie, vaskulärer Demenz amyloidalen Ursprungs, Einschlusskörper-Myositis und Demenz mit Lewy-Körpern.

18. Pharmazeutische Zusammensetzung zur Verwendung nach Anspruch 17, wobei die Amyloidkrankheit die Alzheimer-Krankheit ist.

**Revendications**

1. Composition comprenant un conjugué d'au moins un peptide CysA$\beta$(33-40) (SEQ ID NO : 1) lié à l'hémocyanine de la patelle megathura crenulata (KLH), dans laquelle l'agent de réticulation reliant chaque peptide CysA$\beta$(33-40) à l'hémocyanine de la patelle megathura crenulata (KLH) du conjugué est le N-hydroxysuccinimide ester (SM) de l'acide maléimidobutyrique.

2. Composition pharmaceutique comprenant i) un conjugué comprenant au moins un peptide CysA$\beta$(33-40) (SEQ ID NO : 1) lié à l'hémocyanine de la patelle megathura crenulata (KLH) et ii) un gel d'hydroxyde d'aluminium, dans laquelle l'agent de réticulation reliant chaque peptide CysA$\beta$(33-40) à l'hémocyanine de la patelle megathura crenulata (KLH) du conjugué est le N-hydroxysuccinimide ester (SM) de l'acide maléimidobutyrique, et dans laquelle le pH de la composition se situe dans la plage de 5.8 à 7.0.

3. Composition pharmaceutique selon la revendication 2, dans laquelle le pH de la composition se situe dans la plage de 6.2 à 7.0.

4. Composition pharmaceutique selon la revendication 2, dans laquelle le pH de la composition se situe dans la plage de 5.8 à 6.2.

5. Composition pharmaceutique selon l'une quelconque des revendications 2 à 4, dans laquelle la quantité de peptides CysA$\beta$(33-40) (SEQ ID NO : 1) dans la composition pharmaceutique est d'au moins 100$\mu$g.

6. Composition pharmaceutique selon l'une quelconque des revendications 2 à 4, dans laquelle la quantité de peptides CysA$\beta$(33-40) (SEQ ID NO : 1) dans la composition pharmaceutique est d'au moins 150$\mu$g.

7. Composition pharmaceutique selon l'une quelconque des revendications 2 à 4, dans laquelle la quantité de peptides CysA$\beta$(33-40) (SEQ ID NO : 1) dans la composition pharmaceutique est de 150$\mu$g à 400$\mu$g.

8. Composition pharmaceutique selon l'une quelconque des revendications 2 à 4, dans laquelle la quantité de peptides CysA$\beta$(33-40) (SEQ ID NO : 1) dans la composition pharmaceutique est de 160$\mu$g à 240$\mu$g.

9. Composition pharmaceutique selon l'une quelconque des revendications 2 à 8, dans laquelle les conjugués comprennent à leur tour un rapport d'au moins 45 peptides CysAβ(33-40) (SEQ ID NO : 1) liés à chaque hémocyanine de la patelle megathura crenulata (KLH).

10. Ampoule en verre comprenant la composition pharmaceutique de l'une quelconque des revendications 2 à 9.

11. Procédé pour fabriquer une composition pharmaceutique selon l'une quelconque des revendications 2 à 9, qui comprend les étapes suivantes :

    a. ajouter de l'ester N-hydroxysuccinimide de l'acide maléimidobutyrique (SM) à une composition comprenant de l'hémocyanine de la patelle megathura crenulata (KLH) dans un tampon à un pH compris entre 7.0 et 9.0 ;
    b. éliminer l'excès d'ester N-hydroxysuccinimide de l'acide maléimidobutyrique dans la solution de l'étape a) ;
    c. ajouter des peptides CysAβ(33-40) (SEQ I D NO : 1) dans du DMSO à la solution de l'étape b) à un pH compris entre 6.6 et 7.0 pour produire les conjugués ;
    d. éliminer le peptide libre de la solution de l'étape c) ;
    e. filtrer de façon optionnelle la solution de l'étape d) ;
    f. ajuster le pH de la solution de l'étape d) ou e) à une plage de pH comprise entre 5.8 et 6.2 ; et
    g. ajouter du gel d'hydroxyde d'aluminium à la solution de l'étape f) une fois que le pH a été ajusté.

12. Procédé selon la revendication 11, dans lequel l'excès de l'étape b) est éliminé en utilisant un tampon à 0.02 M de phosphate de sodium à un pH d'environ 6.6 à 7.0.

13. Procédé selon l'une quelconque des revendications 11 ou 12, dans lequel l'excès de l'étape d) est éliminé en utilisant un tampon à 0.01 M de PBS à un pH de 6.6 à 7.0.

14. Procédé selon l'une quelconque des revendications 11 à 13, dans lequel la solution est filtrée dans l'étape e) en utilisant un filtre de 0.2 μm.

15. Procédé selon l'une quelconque des revendications 11 à 14, dans lequel le pH de l'étape f) est ajusté à une plage de pH de 6.0.

16. Composition pharmaceutique de l'une quelconque des revendications 2 à 9 ou ampoule de verre de la revendication 10, destinée à être utilisée dans le traitement ou la prévention d'une maladie amyloïde.

17. Composition pharmaceutique pour une utilisation selon la revendication 16, dans laquelle la maladie amyloïde est choisie dans la liste consistant en la maladie d'Alzheimer, la maladie de Parkinson, l'angiopathie amyloïde cérébrale, la démence vasculaire ayant une origine amyloïde, la myosite à inclusion et la démence à corps de Lewy.

18. Composition pharmaceutique pour une utilisation selon la revendication 17, dans laquelle la maladie amyloïde est la maladie d'Alzheimer.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- ES 2246105
- WO 2005072777 PCT
- US 2013230545 A1
- WO 201501282287 A1

SEQUENCE LISTING

<110> ARACLON BIOTECH, S.L.
<120> Amyloid conjugate and uses and methods thereof
<130> 1500077
<160> 3
<170> PatentIn version 3.5

<210> 1
<211> 9
<212> PRT
<213> Artificial sequence

<220>
<223> Abeta fragment with cysteine added at the N-terminus

<400> 1

```
Cys Gly Leu Met Val Gly Gly Val Val
1               5
```

<210> 2
<211> 40
<212> PRT
<213> Homo sapiens

<400> 2

```
Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val
        35                  40
```

<210> 3
<211> 42
<212> PRT
<213> Homo sapiens

<400> 3

```
Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val Ile Ala
        35                  4
```